# FASCICULE DE BREVET EUROPEEN

(11) **EP 1 768 684 B1**
(45) Date de publication et mention de la délivrance du brevet: **23.05.2012**
(21) Numéro de dépôt: 05775496.2
(22) Date de dépôt: 09.06.2005
(51) Int. Cl.: A61K 36/48, A61K 36/185, A61K 36/66

(54) **COMPOSITION COMPRENANT UN EXTRAIT DE LOTUS BLEU POUR LE TRAITEMENT DES CONTRACTIONS MUSCULAIRES FACIALES INCONTRÔLEES**
ZUSAMMENSETZUNG ENTHALTEND EXTRAKTE VON BLAUEM LOTUS ZUR BEHANDLUNG VON UNKONTROLLIERTEN GESICHTSMUSKELKONTRAKTIONEN
COMPOSITION COMPRISING A BLUE LOTUS EXTRACT FOR THE TREATMENT OF FACIAL UNCONTROLLED MUSCULAR CONTRACTIONS

(30) Priorité: 09.06.2004 FR 0406240; 10.06.2004 FR 0406283; 11.06.2004 FR 0406333
(43) Date de publication de la demande: 04.04.2007
(73) Titulaire: Laboratoire Nuxe, 75010 Paris (FR)
(72) Inventeur: LECLERE, Jacques, F-45500 Saint-Gondon (FR)
(74) Mandataire: L'Helgoualch, Jean
(86) Numéro de dépôt international: PCT/FR2005/001429
(87) Numéro de publication internationale: WO 2006/000708

(56) Documents cités:
- WO-A-2004/017924
- WO-A-2005/077328
- FR-A- 2 805 161
- US-A- 5 925 348
- US-A- 5 985 300
- US-A1- 2002 098 253
- US-B1- 6 296 859
- US-B1- 6 468 564
- PATENT ABSTRACTS OF JAPAN vol. 009, no. 247 (C-307), 3 octobre 1985 (1985-10-03) & JP 60 104005 A (KOBAYASHI KOOSEE:KK), 8 juin 1985 (1985-06-08)
- FOSSEN T ET AL: "Flavonoids from blue flowers of Nymphaea caerulea" PHYTOCHEMISTRY, PERGAMON PRESS, GB, vol. 51, no. 8, août 1999 (1999-08), pages 1133-1137, XP004290945 ISSN: 0031-9422

## Description

La présente invention l'utilisation d' une composition à base d'extrait de lotus bleu, associé le cas échéant à un extrait de coquelicot et/ou un extrait de guimauve, à effet décontractant de la peau, capable d'agir efficacement contre les contractions musculaires faciales incrontrôlées responsables des signes du vieillissement cutané et en particulier les rides d'expression.

La peau constitue un véritable organe comprenant plusieurs couches intégrées, allant de la couche superficielle, l'épiderme, jusqu'au couches plus profondes, le derme et l'hypoderme, et chacune possède des propriétés spécifiques permettant à l'ensemble de réagir et de s'adapter aux conditions de son environnement.

L'épiderme, principalement composé de kératinocytes (90% des cellules épidermiques), de mélanocytes (2 à 3% des cellules épidermiques) et des cellules de Langerhans, a une épaisseur variable selon les différentes parties du corps. Etant donné qu'il constitue la couche externe de la peau, l'épiderme joue un rôle fondamental pour assurer la protection et le maintien d'une bonne trophicité. C'est pourquoi de nombreuses compositions ont été mises au point afin de le protéger et d'améliorer ses fonctions, et notamment de renforcer son élasticité et sa fermeté.

Le derme, plus épais, solide, riche en nerfs, en vaisseaux sanguins et en glandes sudoripares, se compose principalement de collagène, d'élastine et de protéoglycanes. Ces trois types de molécules sont synthétisés par les fibroblastes dermiques. Les fibres de collagène, qui représentent 70% du poids sec du derme, assurent la résistance mécanique et la texture de la peau, l'élastine est responsable de l'élasticité, et les protéoglycanes jouent un rôle majeur de structure et d'hydratation de la peau. D'autres cellules comme les macrophages et les leucocytes sont également présentes dans la couche du derme.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

Le vieillissement de la peau peut être intrinsèque, ou extrinsèque c'est-à-dire provoqué par l'environnement.

L'un des premiers signes de vieillissement de la peau est une perte d'élasticité et la formation de rides et ridules, qui sont le résultat direct de la détérioration de la couche du derme de soutien. L'apparition de marques pigmentaires, la diminution de l'épaisseur de la peau et son affaissement sont également des changements observés au cours du vieillissement.

De nombreux facteurs contribuent à accélérer la dégradation du collagène du derme, et en particulier l'exposition au soleil, les radicaux libres, certains changements hormonaux associés à la vieillesse et le tabac.

Les premiers signes du vieillissement de la peau, tels que les rides et ridules, sont généralement provoqués par le stress et les changements biologiques et physiologiques, accélérés par l'environnement extérieur. En fait, la capacité de la peau à remplacer le collagène endommagé diminue avec le temps, et des espaces et des irrégularités apparaissent dans le réseau du collagène. A l'échelle de la peau, le vieillissement provoque notamment une diminution des synthèses protéiques (collagène, élastine), une diminution de la synthèse des protéoglycanes entre autres, ainsi qu'une élévation des métalloprotéinases de type MMP3.

Ce vieillissement de la peau s'accompagne de sensations de pesanteur, de pression durant le sommeil et aussi de contraction des muscles faciaux, déterminant des rides d'expression, notamment autour des yeux et de la bouche, ou la modification de microrelief épidermique en raison notamment du stress. En particulier, les rides d'expression sont provoquées par de multiples contractions et crispations que subissent quotidiennement les traits du visage sous l'action des muscles sous-cutanés du visage qui se contractent et se relâchent successivement de manière incessante suivant le rythme des émotions.

Pour lutter contre ces signes du vieillissement, il est donc nécessaire de diminuer les forces de contraction des muscles faciaux en exerçant une inhibition de la libération des neuromédiateurs. Ce phénomène d'inhibition a pour conséquence de provoquer une décrispation des traits du visage et de réduire la profondeur des rides faciales.

On connaît certains traitements qui peuvent avoir pour effet de limiter ou d'inhiber les contractions musculaires faciales. Ainsi, on sait qu'il est possible d'effectuer un traitement esthétique par injection de toxine botulique au niveau des rides afin de figer les cellules en contact avec la toxine, donnant à la peau un aspect plus lisse. Certaines compositions cosmétiques contiennent des substances exerçant une action exfoliante comme les alpha-hydroxy acides, par exemple l'acide lactique et l'acide glycolique, facilitant l'élimination des cellules mortes et stimulant la formation de collagène et d'élastine. On connaît aussi des compositions à base d'acide rétinoïque ou de rétinol qui peuvent agir contre les signes du vieillissement cutané tels que la sécheresse, la perte d'élasticité de la peau et la formation de rides, ainsi que des crèmes décontractantes à base de magnésium ou de manganèse inhibant le flux de calcium dans les cellules, à l'origine de la contraction musculaire, afin de réduire les microcrispations de la peau et de ralentir l'apparition des rides d'expression.

Le lotus bleu (Nymphaea caerulea) était déjà utilisé en Egypte antique pour son parfum suave, et sa racine était consommée comme produit alimentaire. Le lotus sacré, qui appartient à une autre espèce, Nelumbo nucifera, différente par son origine et sa composition, contient des enzymes telles que la méthyl transférase présentant un effet anti-âge, comme indiqué dans le brevet US 6.468.564. Le brevet US 5.925.348 décrit une composition cosmétique comprenant aussi un extrait de graines de lotus sacré comme source de méthyl transférase destinée à lutter contre le vieillissement cutané.

Les Nymphaea, et en particulier le lotus, ainsi que les nénuphars contiennent, dans toutes leurs parties, de la nymphéine et de la nupharine (qui sont des alcaloïdes) ainsi que d'autres composants mal définis.

Le coquelicot (Papaver rhoeas) contient de rhoeadine, alcaloïde de la série des tétrahydrobenzazépines, qui est supposée avoir des propriétés neuroleptiques. Cette plante a été décrite comme pouvant être utilisée dans des cas d'éréthisme cardiaque de l'adulte, comme calmant chez les adultes et les enfants, et aussi pour le traitement symptomatique de la taux.

La guimauve (Althea officinalis) est une plante dont plusieurs parties telles que les feuilles et, surtout, les racines, ont depuis longtemps été utilisées, notamment dans des compositions alimentaires ou des suppléments diététiques, mais aussi en raison de propriétés connues. Ainsi, on a observé que la racine de guimauve a une action anti-inflammatoire et calmante sur l'organisme et aussi un effet antitussif qui a justifié son utilisation dans des sirops en pédiatrie. On sait en effet que la racine de guimauve contient un sucre neutre, le rhamnogalacturonane, qui inhibe la toux réflexe provoquée par un stimulus mécanique chez le chat à une dose de 50 mg/kg de poids (G. Nosalova et al. Pharmazie. 1992, 47(3):224-6). La racine de guimauve a aussi été longtemps utilisée comme masticatoire pour calmer les effets des poussées dentaires chez les enfants.

Des extraits de fleurs ou de feuilles de guimauve ont aussi été proposés dans des compositions phytopharmaceutiques destinées à favoriser l'hydratation de la peau, ou encore comme antalgiques pour le traitement des affections de la cavité buccale et du larynx.

Son utilisation comme additif dans des compositions alimentaires ou de confiserie est aussi bien connue. Ainsi le brevet US 6.605.306 décrit un supplément alimentaire contenant divers extraits végétaux dont Althea officinalis pour son action anti-inflammatoire.

Cependant, malgré les diverses compositions disponibles, il existe toujours un besoin de pouvoir disposer de nouvelles compositions topiques alternatives permettant de lutter efficacement contre les effets du vieillissement cutané, et notamment des compositions topiques à base d'extraits végétaux appropriés provenant plus particulièrement de plantes connues pour leurs propriétés favorables.

Suivant la présente invention, on a trouvé qu'il était possible d'agir efficacement contre les contractions musculaires faciales incontrôlées responsables des signes du vieillissement cutané et en particulier les rides d'expression, en utilisant des compositions topiques à base d'extraits de lotus bleu (Nymphaea caerulea), additionnées le cas échéant d'extraits de guimauve (Althea officinalis) et/ou de coquelicot (Papaver rhoeas).

La présente invention a pour objet l'utilisation d'un extrait de lotus bleu (Nymphaea caerulea) pour la préparation d'une composition topique destinée à lutter contre les contractions musculaires faciales incontrôlées.

La présente invention a aussi pour objet l'utilisation d'un extrait de lotus bleu (Nymphaea caerulea), associé à un extrait de guimauve (Althea officinalis) et/ou un extrait de coquelicot (Papaver rhoeas) pour la préparation d'une composition topique destinée à lutter contre les contractions musculaires faciales incontrôlées.

La présente invention a encore pour objet un procédé cosmétique pour combattre les contractions musculaires faciales incontrôlées, consistant à appliquer sur les zones de la peau affectées une composition contenant une quantité efficace de la composition topique selon la présente invention.

L'invention a également pour objet l'utilisation d'un extrait de lotus bleu (Nymphaea caerulea) pour la préparation d'un médicament dermatologique pour la prévention et le traitement des contractions musculaires faciales incontrôlée.

L'invention a également pour objet l'utilisation d.'un extrait de lotus bleu (Nymphaea caerulea) associé à un extrait de guimauve (Althea officinalis) et/ou un extrait de coquelicot (Papaver rhoeas) pour la préparation d'un médicament dermatologique pour la prévention et le traitement des contractions musculaires faciales incontrôlées.

Les compositions suivant la présente invention se distinguent en ce qu'elles comprennent un extrait de lotus bleu (Nymphaea caerulea) en une quantité efficace pour lutter les rides d'expressions, ainsi que des supports et excipients acceptables en dermatologie et en cosmétologie.

Ainsi, les compositions topiques de l'invention peuvent être utilisées avantageusement en dermatologie et en cosmétologie ou réduire les contractions pour prévenir musculaires faciales incontrôlées.

Les extraits végétaux de lotus bleu des compositions suivant la présente invention ont montré dans des tests in vitro des effets d'inhibition des neurotransmetteurs capables de générer les contractions musculaires réflexes, grâce à des neuropeptides dont la modulation est liée à la production d'acétyl choline et d'adrénaline. Les tests ont été réalisés sur une culture de ganglion dorsal de foetus de souris.

Les résultats, détaillés ci-après, ont mis en évidence, selon les concentrations utilisées :
- une diminution jusqu'à 41% de la substance P,
- une diminution jusqu'à 36% de la CGRP (Calcitonine Gene Related Protein),
- une diminution jusqu'à 37% de l'acétyl choline,
- une diminution jusqu'à 35% de l'affinité du récepteur de la substance P au niveau des kératinocytes humaines en culture,
- une inhibition de -24% de la réception de l'adrénaline sur le récepteur adrénergique.

Ces résultats ont été obtenus avec un extrait de lotus bleu (Nymphaea caerulea) et une association d'extraits de lotus bleu et de coquelicot (Papaver rhoeas) éventuellement additionnée d'extrait de guimauve (Althea officinalis), et confirment que, en application topique, un extrait de lotus bleu, ou une composition le contenant, le cas échéant additionnée de coquelicot et/ou de guimauve, présente un effet de relaxation musculaire.

En ce qui concerne le lotus bleu, les essais effectués par la demanderesse ont montré que, parmi les diverses parties de la plante, il est préférable d'utiliser les graines mures, dont l'approvisionnement et la conservation ne soulèvent généralement pas de difficulté technique.

Suivant une forme avantageuse de réalisation, l'extrait de lotus bleu utilisable dans l'invention est obtenu par macération hydro-alcoolique ou hydroglycolique de graines pulvérisées, et de préférence dans un mélange propylène glycol / eau dans un rapport (v/v) voisin de 50/50.

L'extrait se présente sous la forme d'un liquide brun - noir, et se caractérise par :

| | |
|---|---|
| - matières sèches | 2 à 2,5% |
| - densité | 1,019 |
| - indice de réfraction | 1,393 |
| - pH | 6,2 à 6,6 |

Les tests ont été effectués en utilisant des échantillons dosés respectivement à 0,1%, 0,5% et 1% d'extrait.

Les résultats sont explicités à l'Exemple 8 ci-après.

L'extrait de lotus bleu utilisé suivant la présente invention peut être utilisé seul ou en association avec d'autres principes actifs compatibles c'est-à-dire non susceptibles de réagir les uns sur les autres ou de masquer ou limiter leurs effets respectifs.

Suivant une forme préférentielle de réalisation, l'extrait de lotus bleu est associé à un extrait de coquelicot et/ou à un extrait de guimauve.

L'extrait de coquelicot se présente sous la forme d'un liquide jaune pâle, et se caractérise par :

| | |
|---|---|
| - matières sèches | 0,05 à 0,6% |
| - densité | 1,010 à 1,060 |
| - indice de réfraction | 1,375 à 1,395 |
| - pH 6,5 à 7,5Le rapport en poids de l'extrait de coquelicot à l'extrait de lotus bleu peut varier de 1/4 à 4/1 environ et est de préférence voisin de 1/1. Les deux extraits sous forme liquide peuvent être mélangés pour former uncomplexe. | |

L'extrait de guimauve utilisé le cas échéant dans les compositions est de préférence sous forme aqueuse. Suivant une forme avantageuse de réalisation, l'extrait de guimauve est obtenu par lixiviation à partir de racines de guimauve (Althea officinalis) réduites en poudre.

L'extrait de guimauve se présente sous la forme d'un liquide de couleur jaune - ambrée, d'odeur caractéristique, se caractérisant par :

| | |
|---|---|
| - matières sèches | 1,5 à 2% |
| - eau | 98 à 98,5% |
| - densité | 0,993 à 1,00 |
| - indice de réfraction | 1,336 |
| - pH | 6,0 à 6,2 |
| - oses neutres | 839 mg/L |
| - oses acides | 459,8 mg/L |

Les essais effectués par la demanderesse ont montré que, parmi les diverses parties de la plante, il est préférable d'utiliser la racine de guimauve, qui est facilement disponible et dont la conservation ne pose généralement pas de problème.

Lorsqu'ils sont utilisés en combinaison, les extraits de lotus bleu, de coquelicot et de guimauve sont utilisés en quantités équivalentes, c'est-à-dire dans un rapport en poids voisin de 1/1/1. Les extraits peuvent être mélangés sous forme liquide pour former un complexe.

Suivant une forme avantageuse de réalisation, l'extrait de lotus bleu, éventuellement additionné de coquelicot et/ou de guimauve, est complété par des principes actifs ou ingrédients auxiliaires choisis pour leurs propriétés complémentaires, afin de renforcer l'effet d'inhibition des contractions musculaires faciales ou compléter les effets anti-âge de la composition. Ainsi il est particulièrement avantageux de le combiner avec des quantités appropriées de protéines de graines d'amarante (Amarantus caudatus) ou des globulines de pois afin d'obtenir un effet tenseur de la peau, d'huile de Calophylum afin de renforcer l'effet anti-rides, d'Imperata cylindrica, par exemple MOIST 24® de la société Sederma, afin de favoriser l'hydratation de la peau par modification de la pression osmotique, de l'huile d'Echium pour son effet anti-inflammatoire, ou encore un palmitoyl pentapeptide-3 tel que le Matrixyl® ou des dérivés tels que le palmitoyl GHK (possédant la chaîne Glycyl-Histidyl-Lysine) et le palmitoyl GQPR (Glycyl-Glutamyl-Prolyl-Arginine) ou le palmitoyl VGVAPG (Valyl-Glycyl-Valyl-Alanyl-Prolyl-Glycine) associé à un céramide-2, ainsi que d'une manière générale toute association avec un céramide.

Les compositions peuvent se présenter sous toutes les formes galéniques usuelles adaptées à une application topique.

Elles peuvent comprendre entre 0,2% et 10% en poids d'extrait de lotus bleu, tel que défini ci-dessus, par rapport au poids total de la composition, et de préférence entre 0,5 et 5% en poids. Le choix de la dose dans la composition peut être fait en fonction de l'utilisation envisagée. Pour un traitement anti-rides prolongé, on utilise de préférence des doses plus faibles, de l'ordre de 0,5 à 1% tandis qu'un traitement ponctuel peut nécessiter des doses plus élevées.

Lorsqu'elles comprennent un extrait de lotus bleu en association avec un extrait de coquelicot et/ou un extrait de guimauve, les teneurs en chacun de ces extraits peuvent être comme indiqué ci-dessus, et de préférence inférieures à 5% en poids pour chaque extrait.

Les compositions peuvent être présentées sous les formes classiquement utilisées pour une application topique, c'est-à-dire sous forme de gel, lotion, émulsion (en particulier crème ou lait), masque ou pommade, contenant des excipients et supports usuels compatibles et pharmaceutiquement acceptables. Elles peuvent aussi se présenter sous forme de lingettes imbibées d'une solution contenant l'extrait de lotus suivant l'invention, le cas échéant associé à l'extrait de coquelicot et/ou à l'extrait de guimauve. Ces formes d'administration par voie topique sont préparées par les techniques connues, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on préfère utiliser des émulsions à structure lamellaire contenant peu de produits éthoxylés ou n'en contenant pas du tout.

Les compositions topiques utilisées selon l'invention peuvent par exemple comprendre des excipients appropriés pour une administration topique externe, en particulier des excipients acceptables sur le plan dermatologique et cosmétologique. Ces excipients appropriés pour la formulation sont bien connus de l'homme du métier et comprennent en particulier des agents de pénétration tels que l'éthoxydiphénol, le phytantriol, l'octyl dodécanol et l'escine ; les épaississants tels que les gommes naturelles et les polymères de synthèse ; les émollients et les tensioactifs tels que l'octanoate de cétéaryle, le myristate d'isopropyle, l'isononanoate de cétéaryle, la diméthicone, la cyclométhicone, le 3-diisostéarate de polyglycéryle, le polyisobutène hydrogéné, l'alcool cétylique, le palmitate cétylique, le phosphate cétylique ; les émulsifiants ; les conservateurs tels que le phénoxyéthanol, le parahydroxybenzoate de méthyle (méthylparaben), le parahydroxybenzoate d'éthyle (éthylparaben), le parahydroxybenzoate de propyle (propylparaben) et le Phenonip® associant du phénoxyéthanol et des parahydroxybenzoates de méthyle, éthyle, butyle et isobutyle ; les colorants ; les parfums ; etc. D'autres ingrédients peuvent être utilisés dans les compositions : les agents hydratants tels que le propylène glycol, la glycérine, le butylène glycol et également les vitamines antioxydantes telles que la vitamine E, par exemple l'acétate de tocophérol ou le tocotriénol, la vitamine C, les polyphénols naturels. On peut également ajouter à la composition des agents conditionneurs de la peau tels que le nylon et le nitrure de bore.

Les exemples suivants illustrent plus en détail l'invention sans en limiter la portée. Dans tous les exemples de compositions qui suivent, les parties sont exprimées en poids, sauf indication contraire.

### Exemple 1

Suivant les techniques classiques, on prépare une lotion décrispante des muscles faciaux ayant la composition pondérale suivante.

| | |
|---|---|
| Extrait de graines de lotus bleu | 3,0 |
| Glycérine | 5,0 |
| 2-méthyl 1,3-propane diol | 5,0 |
| Phenonip® | 0,5 |
| Panthénol | 0,2 |
| Allantoïne | 0,1 |
| PEG 40 huile de ricin | 1,5 |
| Dipropylène glycol | 1,0 |
| Parfum | 0,5 |
| Eau déminéralisée | qsp 100,0 |

L'extrait de graines de lotus bleu utilisé dans la composition ci-dessus est un extrait hydro-glycolique obtenu par macération de graines pulvérisées dans un mélange propylène glycol / eau 50/50 (v/v) ayant les caractéristiques indiquées plus haut.

La lotion ayant la composition indiquée ci-dessus est utilisée en application sur le visage, une à deux fois par jour.

### Exemple 2

Suivant les techniques classiques, on prépare une crème décrispante des muscles faciaux ayant la composition pondérale suivante.

| | |
|---|---|
| Extrait de graines de lotus bleu | 5,0 |
| Glycérine | 5,0 |
| Phénoxyéthanol | 0,5 |
| EDTA tétrasodique | 0,05 |
| Butylène glycol | 1,0 |
| Carbomer | 0,2 |
| Tocophérol libre | 0,7 |
| Alcool béhénique auto-émulsionnable | 5,0 |
| Octyl dodécanol | 4,0 |
| Palmitate de cétyle | 2,0 |
| Huile de rosier Muscat | 1,0 |
| Trométhamine | 0,2 |
| Matrixyl® | 3,0 |
| Conservateurs | 0,3 |
| Parfum | 0,2 |
| Eau déminéralisée | qsp 100,0 |

L'extrait de graines de lotus bleu utilisé dans la composition ci-dessus est identique à celui de l'exemple 1.

### Exemple 3

On prépare une lingette décrispante des muscles faciaux imbibée d'une lotion ayant la composition pondérale suivante.

| | |
|---|---|
| Extrait de graines de lotus bleu | 5,0 |
| Glycérine | 5,0 |
| PEG 400 | 4,0 |
| Sorbitol à 70% | 2,0 |
| Butylène glycol | 4,0 |
| Phenonip® | 0,5 |
| Parfum hydrosoluble | 1,0 |
| Eau déminéralisée | qsp 100,0 |

### Exemple 4

Suivant les techniques classiques, on prépare une lotion décrispante des muscles faciaux associant des extraits de lotus bleu et de coquelicot, ayant la composition pondérale suivante.

| | |
|---|---|
| Complexe lotus bleu / coquelicot | 3,0 |
| Glycérine | 5,0 |
| 2-méthyl 1,3-propane diol | 5,0 |
| Phenonip® | 0,5 |
| Panthénol | 0,2 |
| Allantoïne | 0,1 |
| PEG 40 huile de ricin | 1,5 |
| Dipropylène glycol | 1,0 |
| Parfum | 0,5 |
| Eau déminéralisée | qsp 100,0 |

Le complexe utilisé dans la composition ci-dessus comprend un extrait de graines de lotus bleu hydro-glycolique et un extrait de coquelicot hydro-glycolique, tous deux obtenus par macération de graines pulvérisées dans un mélange propylène glycol / eau 50/50 (v/v).

La lotion ayant la composition indiquée ci-dessus est utilisée en application sur le visage, une à deux fois par jour.

### Exemple 5

Suivant les techniques classiques, on prépare une crème décrispante des muscles faciaux ayant la composition pondérale suivante.

| | |
|---|---|
| Complexe lotus bleu / coquelicot | 5,0 |
| Glycérine | 5,0 |
| Phénoxyéthanol | 0,5 |
| EDTA tétrasodique | 0,05 |
| Butylène glycol | 1,0 |
| Carbomer | 0,2 |
| Tocophérol libre | 0,7 |
| Alcool béhénique auto-émulsionnable | 5,0 |
| Octyl dodécanol | 4,0 |
| Palmitate de cétyle | 2,0 |
| Huile de rosier Muscat | 1,0 |
| Trométhamine | 0,2 |
| Matrixyl® | 3,0 |
| Conservateurs | 0,3 |
| Parfum | 0,2 |
| Eau déminéralisée | qsp 100,0 |

Le complexe lotus bleu / coquelicot utilisé dans la composition ci-dessus est identique à celui de l'exemple 1.

### Exemple 6

On prépare une lingette décrispante des muscles faciaux imbibée d'une lotion ayant la composition pondérale suivante.

| | |
|---|---|
| Complexe lotus bleu / coquelicot | 5,0 |
| Glycérine | 5,0 |
| PEG 400 | 4,0 |
| Sorbitol à 70% | 2,0 |
| Butylène glycol | 4,0 |
| Phenonip® | 0,5 |
| Parfum hydrosoluble | 1,0 |
| Eau déminéralisée | qsp 100,0 |

### Exemple 7

Suivant les techniques classiques, on prépare une crème décrispante des muscles faciaux ayant la composition pondérale suivante.

| | |
|---|---|
| Extrait de graines de lotus bleu | 2,0 |
| Extrait de coquelicot | 2,0 |
| Extrait de racines de guimauve | |
| (Althea officinalis) | 2,0 |
| Glycérine | 5,0 |
| Phénoxyéthanol | 0,5 |
| EDTA tétrasodique | 0,1 |
| Butylène glycol | 1,0 |
| Carbomer | 0,2 |
| Tocophérol libre | 0,7 |
| Alcool béhénique auto-émulsionnable | 5,0 |
| Octyl dodécanol | 4,0 |
| Palmitate de cétyle | 2,0 |
| Huile de rosier Muscat | 1,0 |
| Trométhamine | 0,2 |
| Matrixyl® | 3,0 |
| Conservateurs | 0,3 |
| Parfum | 0,2 |
| Eau déminéralisée | qsp 100,0 |

Les extraits de lotus bleu, de coquelicot et de guimauve ci-dessus sont utilisés de préférence sous la forme d'un complexe associant les extraits mélangés sous forme liquide.

### Exemple 8

L'étude des effets de l'extrait de lotus bleu suivant la présente invention a été faite sur des ganglions de la racine dorsale de rats, comme indiqué plus haut.

### Protocole expérimental

Les cultures de ganglions de la racine dorsale ont été établies à partir de prélèvements effectués sur des foetus de rat après 14 jours de gestation. Les ganglions qui ont été prélevés le plus stérilement possible, sont conservés dans un tampon sous antibiotique. Les ganglions ont été cultivés dans du milieu DMEM contenant du sérum de veau foetal pendant 17 jours. Pendant cette durée de culture, les ganglions ont émis des prolongements de dendrites qui sécrètent les neuropeptides.

Après 17 jours de culture, le milieu a été éliminé et du milieu frais a été ajouté, soit seul, soit additionné de différentes concentrations de l'extrait suivant l'invention. Les boîtes ont été mises à incuber pendant 24 heures à 37°C dans une étuve à 95% d'oxygène et 5% de CO2.

A la fin de la période d'incubation (24 heures), le milieu de culture a été récupéré. Les neuropeptides (substance P, CGRP, adrénaline et acétylcholine) ont été dosés grâce à un anticorps monoclonal spécifique.

### Dosage de la substance P

Les cultures primaires de cellules nerveuses sont établies à partir de prélèvements effectués au niveau du ganglion dorsal de foetus de souris comme indiqué ci-dessus. Les cellules sont incubées en présence ou en l'absence du produit à l'étude. A la fin du temps d'incubation, le milieu est prélevé, puis la substance P est dosée par réaction immunologique avec des anticorps monoclonaux spécifiques à ce neuropeptide.

L'essai est conduit en triplicate après 24 heures de traitement.
- lot 1 : témoin négatif
- lot 2 : témoin positif (induction par la Capsaïcine)
- lot 3 : traité par le produit Extrait de Lotus Bleu (3 concentrations)
- lot 4 : traité par le complexe Lotus Bleu / coquelicot (2 concentrations)

Les résultats sont regroupés dans le tableau ci-dessous :

| | Substance P (pg/ml) | Pourcentage |
|---|---|---|
| Témoin | 42,8 ± 7,4 | - |
| Témoin positif Capsaicine (25pM) | 64,5 ± 11,4 | +51 |
| Extrait de lotus bleu (0,1%) | 36,8 ± 12** | -14 |
| Extrait de lotus bleu (0,5%) | 31,8 ± 7,8* | -25 |
| Extrait de lotus bleu (1%) | 25,2 ± 8,2* | -41 |
| Complexe coquelicot / lotus (0,5%) | 38,5 ± 10,1 | (ns) |
| Complexe coquelicot / lotus (1%) | 35,8 ± 7,4* | -16 |

| | | |
|---|---|---|
| * Significativement différent par rapport au témoin p≤0,05 (Wilcoxon Rank Sum Test). ** Significativement différent par rapport au témoin p≤0,01 (Wilcoxon Rank Sum Test). | | |

### Dosage de la CGRP

Les cultures primaires de cellules nerveuses sont obtenues de la même façon que pour la substance P et le dosage de la CGRP est fait par réaction immunologique avec des anticorps monoclonaux spécifiques de la CGRP

L'essai est conduit en triplicate après 24 heures de traitement.
- lot 1 : témoin négatif
- lot 2 : témoin positif (induction par la Capsaïcine)
- lot 3 : traité par le produit Extrait de Lotus Bleu (3 concentrations)
- lot 4 : traité par le complexe Lotus Bleu / coquelicot (2 concentrations)

Les résultats sont regroupés dans le tableau ci-dessous :

| | CGRP (pg/ml) | Pourcentage |
|---|---|---|
| Témoin | 290 ± 15,8 | - |
| Témoin positif Capsaicine (25µM) | 345 ± 9,7 | +19 |
| Extrait de lotus bleu (0,1%) | 215 ± 15,8* | -25 |
| Extrait de lotus bleu (0,5%) | 202 ± 22* | -30 |
| Extrait de lotus bleu (1%) | 185 ± 34* | -36 |
| Complexe coquelicot / lotus (0,5%) | 245 ± 11,8** | -15 |
| Complexe coquelicot / lotus (1%) | 223 ± 25,8* | -23 |

| | | |
|---|---|---|
| * Significativement différent par rapport au témoin p≤0,01 (Wilcoxon Rank Sum Test). ** Significativement différent par rapport au témoin p≤0,05 (Wilcoxon Rank Sum Test). | | |

### Dosage de l'acétylcholine

ales cellules nerveuses sont ensemencées comme précédemment. Les cellules sont incubées en présence ou en l'absence de l'extrait de lotus bleu suivant l'invention. A la fin du temps d'incubation, le milieu est prélevé, puis l'acétylcholine est dosée par réaction immunologique avec des anticorps monoclonaux spécifiques.

L'essai est conduit en triplicate après 24 heures de traitements.
- lot 1 : témoin négatif
- lot 2-4 : traité par le produit Extrait de Lotus (3 concentrations)
- lot 5 : traité par le complexe Lotus Bleu / coquelicot (2 concentrations)

Le dosage de l'acétylcholine est déterminé à l'aide du kit AmplexTM Red (Acétylcholine/Acetylcholinesterase) qui fournit une méthode ultrasensible. Les utilisations potentielles de ce kit incluent le criblage des inhibiteurs d'acétylcholine estérase et la mesure de la libération de l'acétylcholine des synaptosomes.

Le dosage de l'acétylcholine est évalué à l'aide d'un lecteur de microplaque en utilisant une excitation à 560 nm et une détection de la fluorescence à 590 nm.

Les résultats sont regroupés dans le tableau ci-dessous :

| | Acétylcholine (µM) | Pourcentage |
|---|---|---|
| Témoin | 23,5 ± 2,8 | - |
| Extrait de lotus bleu (0,1%) | 20,8 ± 0,7 | -11 (ns) |
| Extrait de lotus bleu (0,5%) | 16,7 ± 2,5* | -29 |
| Extrait de lotus bleu (1%) | 14,8 ± 3,2* | -37 |
| Complexe coquelicot / lotus (0, 5%) | 20,5 ± 1,5** | -12 |
| Complexe coquelicot / lotus (1%) | 19,6 ± 2,2** | -16 |

| | | |
|---|---|---|
| * Significativement différent par rapport au témoin p≤0,01 (Wilcoxon Rank Sum Test). ** Significativement différent par rapport au témoin p≤0,05 (Wilcoxon Rank Sum Test). | | |

### Evaluation de l'affinité vis-à-vis du récepteur de la substance P au niveau des kératinocytes humains en culture

Les kératinocytes sont incubés en présence ou en l'absence du produit à l'étude ou de ligand non radioactif (Sar9, Met(O2)11)-SP, avec la 3H (Sar9, Met(O2)11)-SP.

Après incubation, la radioactivité est dosée pour déterminer le potentiel du produit à l'étude à renter en compétition avec la substance P.

L'essai est conduit en triplicate après 24 heures de traitement.
- lot 1 : témoin négatif
- lot 2-4 : traité par le produit Extrait de Lotus Bleu (3 concentrations)
- lot 5-7 : traité par le complexe coquelicot / lotus bleu (3 concentrations)

Cette étude est basée sur la compétition de l'Extrait de Lotus Bleu avec la substance P marquée. Ce résultat se traduit par une diminution de la fixation spécifique de la substance P marquée sur son récepteur quand le produit rentre en compétition.

Les résultats sont regroupés dans le tableau ci-dessous :

| | Cpm | Pourcentage |
|---|---|---|
| Témoin | 1580 ± 124 | - |
| Extrait de lotus bleu (0,1%) | 1205 ± 32* | -23 |
| Extrait de lotus bleu (0,5%) | 1212 ± 102* | -23 |
| Extrait de lotus bleu (1%) | 1113 ± 135* | -30 |
| Complexe coquelicot / lotus (0,1%) | 1113 ± 122* | -29 |
| Complexe coquelicot / lotus (0,5%) | 1123 ± 185* | -29 |
| Complexe coquelicot / lotus (1%)_{.} | 1025 ± 202* | -35 |

| | | |
|---|---|---|
| * Significativement différent par rapport au témoin p≤0,01 (Wilcoxon Rank Sum Test). | | |

Ainsi, les résultats exposés ci-dessus, montrent que le produit testé :
- inhibe la libération de la substance P, aux concentrations 0.5% et 1% respectivement de 25% et 41% par rapport au témoin non traité.
- inhibe la libération de CGRP, aux concentrations 0.5% et 1% respectivement de 30% et 36% par rapport au témoin non traité.
- inhibe la libération de la substance P sur son récepteur aux concentrations 0.1% ; 0,5% et 1% respectivement de 23% et 30%.
- inhibe la libération de l'Acétylcholine, aux concentrations 0.5% et 1% respectivement de 29% et 37% par rapport au témoin non traité.

Ces résultats montrent que le produit testé peut éliminer l'activité de la substance P et la CGRP, en inhibant leur libération et en entrant en compétition avec la substance P sur son récepteur. Cette activité au niveau des neuropeptides et plus précisément l'inhibition de leur libération peut être due à l'inhibition de l'Acétylcholine.

En effet il a été montré que l'acétylcholine peut activer les fibres C nociceptives avec, pour conséquence, une augmentation de la concentration du CGRP et de la substance P.

### Dosage de l'adrénaline

Les résultats obtenus après traitement des mêmes cellules que dans les essais ci-dessus par l'extrait de lotus bleu de l'invention montrent une compétition de l'extrait avec l'adrénaline vis-à-vis du récepteur adrénergique. Cette fixation sur le récepteur adrénergique peut réguler le mécanisme de la contractilité via la calmoduline. L'affinité du complexe calcium - calmoduline pour l'ATPase de la myosine dépend du degré de phosphorylation d'une kinase des chaînes légères de la myosine. Lorsque celle-ci est phosphorylée sous l'action de la protéine kinase A, l'affinité du complexe calcium - calmoduline pour l'ATPäse de la myosine diminue, aboutissant à une relaxation des fibres musculaires.

Les résultats sont regroupés dans le tableau ci-dessous :

| | [¹²⁵I] Adrénaline (Cpm) | Pourcentage |
|---|---|---|
| Témoin | 3850 ± 270 | - |
| Extrait de lotus bleu (0,1%) | 3415 ± 211 | -11 |
| Extrait de lotus bleu (0,5%) | 3218 ± 198* | -16 |
| Extrait de lotus bleu (1%) | 2915 ± 153* | -24 |

| | | |
|---|---|---|
| * Significativement différent par rapport au témoin p≤0,05 (Wilcoxon Rank Sum Test). | | |

Ces résultats mettent en évidence l'effet relaxant des fibres musculaires exercé par l'extrait de lotus bleu de l'invention.

## Revendications

1. Utilisation d'un extrait de lotus bleu (Nymphaea caerulea) pour la préparation d'une composition topique pour la prévention et le traitement des contractions musculaires faciales incontrôlées.

2. Utilisation selon la revendication 1, **caractérisée en ce que** l'extrait est obtenu à partir de graines de lotus bleu (Nymphea caerulea).

3. Utilisation selon la revendication 2, **caractérisée en ce que** l'extrait est obtenu par macération hydro-glycolique de graines pulvérisées.

4. Utilisation selon la revendication 3, **caractérisée en ce que** l'extrait est obtenu par macération de graines pulvérisées dans un mélange propylène glycol / eau.

5. Utilisation selon l'une quelconque des revendications précédents, **caractérisée en ce que** l'extrait de lotus bleu (Nymphaea caerulea) est un liquide brun - noir **se caractérisant par** :
| | |
|---|---|
| - matières sèches | 2 à 2,5% |
| - densité | 1,019 |
| - indice de réfraction | 1,393 |
| - pH | 6,2 à 6,6 |

6. Utilisation selon l'une quelconque des revendications précédentes, **caractérisée en ce que** la composition comprend entre 0,2 et 10% en poids d'extrait de lotus bleu.

7. Utilisation selon l'une quelconque des revendications précédentes, **caractérisée en ce que** la composition comprend en outre un extrait de coquelicot (Papaver rhoeas) et/ou un extrait de guimauve (Althea officinalis).

8. Utilisation selon la revendication 7, **caractérisée en ce que** l'extrait de coquelicot (Papaver rhoeas) se présente sous la forme d'un liquide jaune pâle, **se caractérisant par** :
| | |
|---|---|
| - matières sèches | 0,05 à 0,6% |
| - densité | 1,010 à 1,060 |
| - indice de réfraction | 1,375 à 1,395 |
| - pH | 6,5 à 7,5 |

9. Utilisation selon la revendication 7, **caractérisée en ce que** l'extrait de guimauve (Althea officinalis) se présente sous la forme d'un liquide de couleur jaune - ambrée, d'odeur caractéristique, **se caractérisant par** :
| | |
|---|---|
| - matières sèches | 1,5 à 2% |
| - eau | 98 à 98,5% |
| - densité | 0,993 à 1,00 |
| - indice de réfraction | 1,336 |
| - pH | 6,0 à 6,2 |
| - oses neutres | 839 mg/L |
| - oses acides | 459,8 mg/L |

10. Utilisation selon l'une quelconque des revendications 1 à 8, **caractérisée en ce que** la composition comprend un extrait de lotus bleu et un extrait de coquelicot dans un rapport en poids compris entre 1/4 et 4/1.

11. Utilisation selon l'une quelconque des revendications 1 à 9, **caractérisée en ce que** la composition comprend un complexe constitué par un mélange d'extraits de lotus bleu, de coquelicot et de guimauve sous forme liquide.

12. Utilisation selon l'une quelconque des revendications précédentes, **caractérisée en ce que** la composition comprend en outre des protéines de graines d'amarante (Amarantus caudatus) ou des globulines de pois, de l'huile de Calophylum, de l'Imperata cylindrica, de l'huile d'Echium, un palmitoyl pentapeptide-3 ou des dérivés tels que le palmitoyl GHK et le palmitoyl GQPR ou le palmitoyl VGVAPG associé à un céramide-2, ainsi qu'un céramide.

13. Utilisation d'un extrait de lotus bleu (Nymphaea caerulea) pour la préparation d'un médicament dermatologique pour la prévention et le traitement des contractions musculaires faciales incontrôlées.

14. Utilisation d'un extrait de lotus bleu (Nymphaea caerulea) associé à un extrait de guimauve (Althea officinalis) et/ou un extrait de coquelicot (Papaver rhoeas) pour la préparation d'un médicament dermatologique pour la prévention et le traitement des contractions musculaires faciales incontrôlées.

## Claims

1. The use of a blue lotus *(Nymphaea caerulea)* extract for preparing a topical composition for preventing and treating facial uncontrolled muscular contractions.

2. The use according to claim 1, **characterized in that** the extract is obtained from blue lotus *(Nymphaea caerulea)* seeds.

3. The use according to claim 2, **characterized in that** the extract is obtained by hydroglycolic maceration of pulverized seeds.

4. The use according to claim 3, **characterized in that** the extract is obtained by maceration of pulverized seeds in a propylene glycol/water mixture.

5. The use according to any of the preceding claims, **characterized in that** the blue lotus *(Nymphaea caerulea)* extract is a brown-black liquid, **characterized by**:
| | |
|---|---|
| - dry materials | 2-2.5% |
| - density | 1.019 |
| - refractive index | 1.293 |
| - pH | 6.2-6.6 |

6. The use according to any of the preceding claims, **characterized in that** the composition comprises between 0.2 and 10% by weight of blue lotus extract.

7. The use according to any of the preceding claims, **characterized in that** the composition further comprises a poppy *(Papaver rhoeas*) extract and/or a marsh mallow *(Althea officinalis)* extract.

8. The use according to claim 7, **characterized in that** the poppy *(Papaver rhoeas)* extract appears as a pale yellow liquid, **characterized by**:
| | |
|---|---|
| - dry materials | 0.05-0.6% |
| - density | 1.010-1.060 |
| - refractive index | 1.375-1.395 |
| - pH | 6.5-7.5 |

9. The use according to claim 7, **characterized in that** the marsh mallow *(Althea officinalis)* extract appears as a liquid of yellow-amber color, with a characteristic odor, **characterized by**:
| | |
|---|---|
| - dry materials | 1.5-2% |
| - water | 98-98.5% |
| - density | 0.993-1.00 |
| - refractive index | 1.336 |
| - pH | 6.0-6.2 |
| - neutral oses | 839 mg/L |
| - acid oses | 459.8 mg/L |

10. The use according to any of claims 1 to 8, **characterized in that** the composition comprises a blue lotus extract and a poppy extract in a weight ratio comprised between 1/4 and 4/1.

11. The use according to any of claims 1 to 9, **characterized in that** the composition comprises a complex formed by a mixture of blue lotus, poppy and marsh mallow extracts in liquid form.

12. The use according to any of the preceding claims, **characterized in that** the composition further comprises proteins from love-lies-bleeding *(Amaranthus caudatus)* seeds or pea globulins, *Calophylum, Imperata cylindrica* oil, *Echium* oil, a palmitoyl pentapeptide-3 or derivatives such as palmitoyl GHK and palmitoyl GQPR or palmitoyl VGVAPG associated with a ceramide-2, as well as a ceramide.

13. The use of a blue lotus *(Nymphaea caerulea)* extract for preparing a dermatological drug for preventing and treating facial uncontrolled muscular contractions.

14. The use of a blue lotus *(Nymphaea caerulea)* extract associated with a marsh mallow *(Althea officinalis)* extract and/or poppy *(Papaver rhoeas)* extract for preparing a dermatological drug for preventing and treating facial uncontrolled muscular contractions.

## Patentansprüche

1. Verwendung eines Extrakts aus blauem Lotus (Nymphaea caerulea) für die Zubereitung einer topischen Zusammensetzung zur Vorbeugung und Behandlung von unkontrollierten Gesichtsmuskelkontraktionen.

2. Verwendung nach Anspruch 1, **dadurch gekennzeichnet, dass** der Extrakt aus Samen des blauen Lotus (Nymphea caerulea) gewonnen wird.

3. Verwendung nach Anspruch 2, **dadurch gekennzeichnet, dass** der Extrakt durch Hydroglykol-Mazeration der pulverisierten Samen gewonnen wird.

4. Verwendung nach Anspruch 3, **dadurch gekennzeichnet, dass** der Extrakt durch Mazeration pulverisierter Samen in einem Propylenglykol-Wasser-Gemisch gewonnen wird.

5. Verwendung nach einem der vorangehenden Ansprüche, **dadurch gekennzeichnet, dass** der blaue Lotusextrakt (Nymphaea caerulea) eine braunschwarze Flüssigkeit ist, die **gekennzeichnet ist durch**:
| | |
|---|---|
| - Trockenmasse | 2 bis 2,5 % |
| - Dichte | 1,019 |
| - Refraktionsindex | 1,393 |
| - pH | 6,2 bis 6,6 |

6. Verwendung nach einem der vorangehenden Ansprüche, **dadurch gekennzeichnet, dass** die Zusammensetzung zwischen 0,2 und 10 Gew.% blauen Lotusextrakt umfasst.

7. Verwendung nach einem der vorangehenden Ansprüche, **dadurch gekennzeichnet, dass** die Zusammensetzung ferner einen Klatschmohnextrakt (Papaver rhoeas) und/oder einen Eibischextrakt (Althea officinalis) umfasst.

8. Verwendung nach Anspruch 7, **dadurch gekennzeichnet, dass** der Klatschmohnextrakt (Papaver rhoeas) die Form einer blassgelben Flüssigkeit hat, die **gekennzeichnet ist durch**:
| | |
|---|---|
| - Trockenmasse | 0,05 bis 0,6 % |
| - Dichte | 1,010 bis 1,060 |
| - Refraktionsindex | 1,375 bis 1,395 |
| - pH | 6,5 bis 7,5 |

9. Verwendung nach Anspruch 7, **dadurch gekennzeichnet, dass** der Eibischextrakt (Althea officinalis) die Form einer bernsteingelben Flüssigkeit mit einem charakteristischen Geruch hat, die **gekennzeichnet ist durch**:
| | |
|---|---|
| - Trockenmasse | 1,5 bis 2 % |
| - Wasser | 98 bis 98,5 % |
| - Dichte | 0,993 bis 1,00 |
| - Refraktionsindex | 1,336 |
| - pH | 6,0 bis 6,2 |
| - neutrale Monosaccharide | 839 mg/L |
| - saure Monosaccharide | 459,8 mg/L |

10. Verwendung nach einem der Ansprüche 1 bis 8, **dadurch gekennzeichnet, dass** die Zusammensetzung einen blauen Lotusextrakt und einen Klatschmohnextrakt in einem Gewichtsverhältnis zwischen 1/4 und 4/1 umfasst.

11. Verwendung nach einem der Ansprüche 1 bis 9, **dadurch gekennzeichnet, dass** die Zusammensetzung einen Komplex umfasst, die von einem Gemisch aus blauen Lotos-, Klatschmohn- und Eibischextrakten in flüssiger Form gebildet wird.

12. Verwendung nach einem der vorangehenden Ansprüche, **dadurch gekennzeichnet, dass** die Zusammensetzung ferner Proteine aus Amarant-Samen (Amarantus caudatus) oder Erbsenglobuline, Calophyllumöl, Imperata cylindrica, Echiumöl, ein Palmitoylpentapeptid-3 oder Derivate wie das Palmitoyl GHK und das Palmitoyl GQPR oder das Palmitoyl VGVAPG in Kombination mit einem Ceramid-2, sowie ein Ceramid umfasst.

13. Verwendung eines blauen Lotusextrakts (Nymphaea caerulea) für die Zubereitung eines dermatologischen Arzneimittels zur Vorbeugung und Behandlung von unkontrollierten Gesichtsmuskelkontraktionen.

14. Verwendung eines blauen Lotusextrakts (Nymphaea caerulea) in Kombination mit einem Eibischextrakt (Althea officinalis) und/oder einem Klatschmohnextrakt (Papaver rhoeas) für die Zubereitung eines dermatologischen Arzneimittels zur Vorbeugung und Behandlung von unkontrollierten Gesichtsmuskelkontraktionen.
